# EUROPÄISCHE PATENTSCHRIFT

(11) **EP 1 761 290 B1**
(45) Veröffentlichungstag und Bekanntmachung des Hinweises auf die Patenterteilung: **19.03.2008**
(21) Anmeldenummer: 05768123.1
(22) Anmeldetag: 23.06.2005
(51) Int. Cl.: A61M 5/00, A61M 5/145, G01R 33/28

(54) **HYDRAULISCHES INJEKTIONSSYSTEM UND VERFARHREN ZUR INJEKTION**
HYDRAULIC INJECTION SYSTEM AND INJECTION METHOD
SYSTEME D'INJECTION HYDRAULIQUE, ET PROCEDE D'INJECTION

(30) Priorität: 24.06.2004 DE 102004030690
(43) Veröffentlichungstag der Anmeldung: 14.03.2007
(73) Patentinhaber: E-Z-EM, Inc., Lake Success, NY 11042 (US)
(72) Erfinder: E-Z-EM, Inc., Lake Success, NY 11042 (US)
(74) Vertreter: Korenberg, Alexander Tal
(86) Internationale Anmeldenummer: PCT/EP2005/006809
(87) Internationale Veröffentlichungsnummer: WO 2006/000415

(56) Entgegenhaltungen:
- US-A- 4 250 887
- US-A- 5 494 036
- US-A1- 2002 115 933

## Beschreibung

Die Erfindung betrifft ein Injektionssystem zur Injektion von Flüssigkeiten innerhalb eines starken Magnetfelds, eines magnetischen Wechselfelds und/oder eines elektrischen Hochfrequenzfelds zur Verwendung mit einem medizinisch-technischen System mit einem gegen elektromagnetische Felder mittels einer Abschirmung abgeschirmten Raum, einer Injektionsvorrichtung, durch die zu injezierende Flüssigkeit in einen Patienten abgebbar ist, einer Antriebseinrichtung der Injektionsvorrichtung, mit der mindestens ein Förderelement verlagerbar ist und einer außerhalb des Raumes angeordneten Steuer- und Kontrolleinheit.

Derartige Injektionssysteme werden insbesondere im Bereich der Magnet-Resonanz-Tomografie (MRT) verwendet, um durch Injektion eines Kontrastmittels diagnosefähige Tomogramme von inneren Organen eines Patienten zu erhalten. Es ist allgemein bekannt, dass MRT-Systeme bestmöglich von äußeren elektromagnetischen Feldern isoliert werden müssen, um eine optimale Bild- bzw. Tomogrammqualität durch das MRT-System zu erhalten. Erreicht wird dies dadurch, dass der gesamte Raum, in dem sich das MRT-System befindet, durch ein Kupferschild oder durch leitfähiges Maschenmaterial vor derartigen z. T. unvorhersagbaren Quellen elektromagnetischer Felder abgeschirmt wird. Um den Vorteil eines derartig abgeschirmten. Raumes voll auszunutzen, wird weiterhin versucht, auch das Injektionssystem und Verfahren zur Injektion von Flüssigkeiten, insbesondere also von Kontrastmedien und Kochsalzlösung zur Verwendung mit einem MRT-System, gegenüber dem MRT-System abzuschirmen oder mindestens zum Teil nach außen zu verlagern, da auch das Injektionssystem und - verfahren selbst Quelle elektromagnetischer Felder sein und die Bildqualität negativ beeinflussen kann.

Ein Injektionssystem der eingangs beschriebenen Art ist beispielsweise aus der US 5,494,036 A allgemein bekannt. Dieses System verhindert durch umfangreiche konstruktive Maßnahmen eine zu starke Beeinflussung der Bildqualität durch elektromagnetische Strahlung.

Die Injektionsvorrichtung, welche in der US 5,494,036 A offenbart wird, besteht aus einer Antriebseinrichtung in Form zweier DC-Elektromotoren und zwei Zylinder-Kolben-Einheiten in Form von Spritzen. Über eine Kopplungseinrichtung, die optional auch hydraulisch ausgestaltet sein kann, die insbesondere jedoch durch zwei Antriebsspindeln (flexible drive shaft) gebildet ist, werden die Kolben der Spritzen mittels der Antriebsspindeln vor und zurück voneinander unabhängig verlagert. Dabei wird eine Drehbewegung eines ein Gewinde aufweisenden, axial feststehenden Antriebselements des jeweiligen Elektromotors in eine lineare Vor- und Rückwärtsbewegung des Spritzenkolbens in der Spritze umgesetzt. Durch die Vor- und Rückwärtsbewegung des Spritzenkolbens kann Kontrastmittel bzw. Kochsalzlösung aufgezogen und später abgegeben werden. Die Spritzen sind über einen Y-Adapter und Zwischenschläuche an einen Katheter anschließbar, durch den das Kontrastmittel und die Kochsalzlösung dem Patienten während des Tomografievorgangs injezierbar ist.

Die Elektromotoren sind über abgeschirmte Elektrokabel an eine Batterie angeschlossen, die sich innerhalb des Raumes befindet. Die Elektromotoren werden insbesondere von einer in dem Raum angeordneten lnjektionskontrolleinheit gesteuert, welche mit einem im abgeschirmten Raum befindlichen Sende- und Empfangsgerät verbunden ist. Mit einem sich außerhalb des Raumes befindlichen weiteren Sende- und Empfangsgerät ist die übergeordnete Steuer- und Kontrolleinheit verbunden. Über die beiden Sende- und Empfangsgeräte steuert und kontrolliert die Steuer- und Kontrolleinheit die Injektionskontrolleinheit.

Ein Nachteil eines derartigen Systems ist, dass durch die Batterie, das Sende- und Empfangsgerät sowie insbesondere die beiden Elektromotoren und die lnjektionskontrolleinheit mögliche Quellen elektromagnetischer Felder vorhanden sind, welche die mit erheblichem Aufwand erreichte Qualität der MR-Tomografie vermindern können. Zur Vermeidung dieser potentiellen Störquellen werden deswegen vor allem nicht elektrisch leitende Materialien verwendet und eine aufwändige Abschirmung aller erwähnten Objekte und deren Zuleitungen und Verbindungsleitungen vorgenommen. Auch muss aus oben genannten Gründen stets ein ausreichender Mindestabstand der Objekte zum Magneten während der Tomografie eingehalten werden, womit ein gewisses Risiko einer mangelhaften Tomogrammerstellung bei der unabsichtlichen Unterschreitung des Mindestabstandes durch das Fachpersonal einhergeht. Ebenfalls nachteilig ist das Gewicht der Objekte, insbesondere der Batterie, sowie deren Platzbedarf. Angesichts der hohen Kosten der Raumabschirmung ist der für das bekannte System benötigte Platz sehr teuer. Schließlich ist von Nachteil, dass der schnelle unbehinderte Zugang zum Patienten und/oder Magneten durch das bekannte System durch vorhandene Stativ- oder Rollwagenaufbauten, auf denen das bekannte System zum Teil installiert ist, sowie durch über den Boden verlaufende Kabelleitungen erschwert wird.

Aufgabe der Erfindung ist es, alle oben beschriebenen Nachteile zu minimieren oder gänzlich abzuschaffen.

US-A-2002/115933 offenbart ein Injektionssystem mit einer Druckerzeugungseinheit in Form einer Pumpe, deren Förderstrom unter anderem über Volumenstrommesser einem Druckumsetzer in Form einer Kolben-Zylinder-Einheit in einem Patientenraum zugeführt wird.

Die erfindungsgemäße Aufgabe wird gelöst durch eine hydraulische Antriebseinrichtung nach Anspruch 1 deren mindestens eine Hydraulikleitung zu einer Druckerzeugungseinheit geführt ist.

Durch die erfindungsgemäße Verwendung von Hydraulik-Komponenten werden bisher verwendete Baugruppen wie Batterie, Elektromotoren, Stromkabel, Injektionskontrolleinheit sowie die beiden Sende- und Empfangsgeräte innerhalb des Raumes überflüssig. Insbesondere entstehen durch die Verwendung des Injektionssystems nach der Erfindung keinerlei ungewünschte elektromagnetische Felder, da für das Injektionssystem kein Strom und kein leitfähiges Material innerhalb des Raumes verwendet werden braucht. Ebenfalls von Vorteil ist, dass die Injektionseinrichtung im Vergleich zu bekannten Injektionseinrichtungen direkt neben dem Patienten gelagert sein kann und selbst in die MRT-Röhre eingefahren werden kann, ohne die Tomografie-Resultate negativ zu beeinflussen. Dadurch lässt sich die Länge der Schläuche über die das Kontrastmittel von der Injektionseinrichturig zur Injektionsnadel geführt wird auf ein noch leicht handhabbares Minimum reduzieren. Durch die verwendbaren kurzen Schläuche ist es insbesondere möglich, Kontrastmittel einzusparen, welches in den Schläuchen vor der Injektion blasenfrei vorliegen muss. Dadurch lässt sich eine erhebliche Kostenreduktion erreichen und vor allem die Patentensicherheit durch eine vereinfachte Prüfung der kürzeren Schlauchverbindungen auf Blasenfreiheit noch weiter erhöhen.

Während es also nach der Erfindung grundsätzlich möglich ist, die Druckerzeugungseinheit sowohl innerhalb als auch außerhalb des Raumes anzuordnen, bietet die Platzierung außerhalb besonders gute Möglichkeiten, elektromagnetische Störeinflüsse zu vermeiden. Bei einer Anordnung der Druckerzeugungseinheit innerhalb des Raumes sollte sich diese vorzugsweise in größtmöglicher Entfernung vom bildgebendem System befinden, z.B. in einer Raumecke mit Abstand von ca. 3 bis 5 m vom bildgebendem System (z.B. MR-Röhre).

Erfindungsgemäß dienen die aus dem Raum heraus zu einer Druckerzeugungseinheit geführten Hydraulikleitungen bzw. die in ihnen geführte Hydraulikflüssigkeit zugleich als direkte oder indirekte Antriebsmittel für ein Förderelement und als Informationsträger, indem hydraulische Parameter wie Druck, Druckänderung, Durchflussmenge und andere Parameter wie Verschiebung der Hydraulikflüssigkeit innerhalb der Leitungen bzw. der daran angeschlossenen Einheiten, von der Hydraulikflüssigkeit selbst zu der Druckerzeugungseinheit übertragen werden und hier leicht auf direkte oder indirekte, an sich bekannte Weise außerhalb des Raumes gemessen werden können. Wesentlich ist der Durchtritt der Hydraulikleitungen durch die Abschirmung des Raumes, da außerhalb desselben keine besonderen Anforderungen an die magnetischen Eigenschaften eventueller weiterer Komponenten der Antriebseinrichtung, insbesondere der Druckerzeugungseinheit, zu stellen sind. Selbstverständlich lassen sich die positiven Eigenschaften der Hydraulik grundsätzlich auch auf die Pneumatik übertragen, in dem pneumatische statt hydraulische Betriebsmittel verwendet werden.

Da bei Verwendung von Hydraulikleitungen die Lage und das Potenzial von elektromagnetischen Feldern nicht berücksichtigt werden muss, ist es zudem in anderen entsprechend sensitiven medizinisch-technischen Systemen vorteilhaft, Hydraulikleitungen zu verwenden. Bei einem Kern-Spin-Tomografiegerät lässt sich beispielsweise die Hydraulikleitung hervorragend in einem Ableitungsschacht des MR-Tomografiegeräts integrieren.

Die Erfindung weiter ausgestaltend, wird vorgeschlagen, dass die Druckerzeugungseinheit in die Steuer- und Kontrolleinheit integriert ist, um den Platzbedarf zu reduzieren und doppelt vorhandene Baugruppen, wie etwa solche zur Erzeugung der Betriebsspannung einzusparen. Auch kann als Antrieb für die außerhalb des Raumes angeordnete Druckerzeugungseinheit ohne weiteres ein Elektromotor verwendet werden, ohne diesen besonders abzuschirmen.

Weiterhin wird vorgeschlagen, dass das Injektionssystem innerhalb des Raumes aus im wesentlichen elektrisch nicht leitfähigem Material besteht, um keine elektromagnetischen . Störquellen innerhalb des Raumes zu erzeugen. Der bewusste Verzicht auf aktive Bauelemente des Injektionssystems in der Nähe des bildgebenden Feldes und die damit einhergehende Abwehr von Gefahren, wie artifizielle magnetische oder elektrische Störfelder, die das Tomografieergebnis negativ beeinflussen können, stellt einen weiteren Vorteil dar. Direkte Gefahren von durch magnetische Anziehung beschleunigten Elementen des Systems werden bei Aufstellung außerhalb des Raumes gänzlich vermieden, da kein Element des Injektionssystems innerhalb des Raums magnetisierbar sein muss bzw. ist

Die Erfindung kann auch dadurch vorteilhaft ausgestaltet werden, dass die Hydraulikleitungen innerhalb des Raumes mindestens zu einem Teil abgedeckt in und/oder auf dem Boden, der Wand und/oder der Decke verlaufen, um so dem Fachpersonal beim Arbeiten mit dem medizinisch-technischen System, insbesondere mit dem MRT-System, ein sicheres und flexibles Betreten des abgeschirmten Raumes zu ermöglichen.

Ebenfalls kann eine Weiterbildung der Erfindung darin bestehen, dass die Hydraulikleitungen mit medizinisch verträglichem also biokompatiblen Hydraulikflüssigkeit befüllt sind, um bei einer Leckage der Leitungen und etwaigem Kontakt der Hydraulikflüssigkeit mit dem Patienten die Gefahr von Hautreizung oder anderen eventuellen Schädigungen völlig auszuschließen.

Ebenfalls soll die Erfindung noch vorteilhaft dadurch ausgestaltet werden, dass die Hydraulikflüssigkeit unter einem Ruhedruck von 0,5 bis 2 bar in den Hydraulikleitungen vorliegt. Durch ständige Drucküberwachung innerhalb oder außerhalb der Druckerzeugungseinheit kann so gewährleistet werden, dass ein durch eine Leckage auftretender Druckabfall sofort detektierbar ist. Dieser könnte z.B. aus Sicherheitsgründen zu einer sofortigen Abschaltung des gesamten Infusionssystems genutzt werden.

Die Erfindung weiter ausgestaltend ist vorgesehen, dass ein Druckumsetzer von zwei Druckerzeugungseinheiten versorgbar ist, von denen je eine mit einem Antrieb verbunden ist. Dabei kann durch die Steuerung und gleichzeitige Messung der jeweiligen Antriebsparameter eines Antriebs der jeweils andere Antrieb überwacht werden, da der durch den Druckumsetzer umgesetzte Druck den Zustand eines Antriebs dem anderen Antrieb direkt übermittelt und vice versa. Zum Beispiel kann, falls der Antrieb als Elektromotor ausgestaltet ist, über eine Strommessung des Motors, die fast linear proportional zum Kraftaufwand für die Aufbringung des Hydraulikdrucks ist, der Zustand des anderen Motors bei gleichzeitiger Direktmessung des Drucks in der Hydraulikleitung ermittelt werden, wenn bei diesem ebenfalls der Strom gemessen und ausgewertet wird. Dabei können die Antriebe sogar gegeneinander arbeiten, wobei dann natürlich eine gewisse Leistungsdifferenz zwischen beiden Antrieben für eine Injektion vonnöten wäre.

Ein weiterer Vorteil ist, dass der Druckumsetzer eine Kolben-Zylinder-Einheit mit zwei Arbeitsräumen aufweist, die durch einen an das Abtriebselement gekoppelter Kolben voneinander getrennt werden. Das im Grunde bekannte Hydraulikprinzip des doppelt wirkenden Kolbens kann somit auch im medizinisch-technischen Bereich energie-effizient, kostengünstig, und hervorragend steuerbar eingesetzt werden.

Im Gegensatz zu bekannten Injektionseinrichtungen, welche auf Stativen oder ähnlichem aufgestellt sind und einen erheblichen Platz benötigen, lässt sich die erfindungsgemäß sehr leichte Antriebseinrichtung direkt an der in der Regel nur wenig belastbaren MRT-Röhre, insbesondere durch einfaches Klettband, befestigen.

Ein Vorteil einer erfindungsgemäßen Antriebseinrichtung ist deren leicht zu bewerkstelligende mechanische Verriegelbarkeit etwa durch Raststangen, Gewindestangen oder andere im Grunde bekannte mechanische Ausführungsformen. Aus Sicherheitsgründen kann das Injektionssystem auf diese einfache Weise sehr schnell zum Stoppen gebracht werden.

Das Verfahren zur Injektion von Flüssigkeit in einen Patienten während einer Magnet-Resonanz-Tomografie durch ein MRT-System ist durch die Nutzung von hydraulischen Parametern wie Druck, Druckänderung pro Zeiteinheit, Durchflussrate, Flüssigkeitsvolumen etc. als Überwachungs- bzw. Steuerungsgrößen ebenfalls von Vorteil, da durch die hydraulische lnformationsübermittlung nach außen auf eine komplexe elektronische Überwachung im Rauminneren verzichtet werden kann.

Ebenfalls günstig ist, dass die Druckerzeugungseinheit durch ein Steuersignal einer Fernbedienung, die sich im Raum befindet, gesteuert werden kann, wobei die Fernbedienung unidirektional Steuersignale aussendet und eine Empfangseinheit innerhalb der Raumes die Steuersignale empfängt und über eine Versorgungsleitung in das elektrische Netz einspeist, da derartige Geräte relativ kostengünstig produzierbar sind. Dabei ist vorzugsweise vorgesehen, dass die Steuersignale Infrarot-Signale sind und die Übertragung durch ein Fenster des Raums oder durch Modulation der Steuersignale auf eine Netzfrequenz durch die Empfangseinheit, welche einer Steckdose einsteckbar ist, stattfindet.

Gemäß einer anderen Ausgestaltung des Injektionssystems ist erfindungsgemäß vorgesehen, dass die Druckerzeugungseinheit eine Hydraulikpumpe bzw. die Druckerzeugungseinrichtung eine solche enthält. Auf diese Weise lässt sich eine einzige Druckerzeugungseinheit für eine Mehrzahl von Druckumsetzern verwenden, wobei die Zuteilung der unter Druck stehenden Hydraulikflüssigkeit zu den einzelnen Druckumsetzern bedarfgerecht erfolgt.

Die Zuteilung des jeweils benötigten Volumenstroms der Hydraulikflüssigkeit zu den einzelnen Druckumsetzern erfolgt in besonders vorteilhafter Weise über jeweils ein Proportionalventil, das eine Verbindung zwischen der Druckseite der Hydraulikpumpe und dem jeweiligen Druckumsetzer herstellt. Besonders vorteilhaft ist es, wenn das Proportionalventil mit beiden Arbeitsräumen des zugeordneten Druckumsetzers verbunden ist. Auf diese Weise lassen sich Vorwärts- und Rückwärtsbewegungen des Kolbens des jeweiligen Druckumsetzers mittels desselben Proportionalventils steuern.

In dem verwendeten Hydrauliksystem sollte des Weiteren ein auf der Druckseite der Hydraulikpumpe angeordneter Druckspeicher vorhanden sein sowie ebenfalls ein Drucksensor, mit dem Druck auf der Druckseite der Hydraulikpumpe messbar und die Hydraulikpumpe bei Überschreiten eines oberen Grenzdrucks abschaltbar und bei Unterschreiten eines unteren Grenzdrucks einschaltbar ist Auf diese Weise ist stets eine gewisse Druckreserve in dem Hydrauliksystem vorhanden, so dass auch bei Stillstand der Pumpe unmittelbar die Verstellbewegung des Kolbens des Druckumsetzers beginnen kann. Sobald ein hinreichend großer Druckabfall in dem Hydrauliksystem auftritt, beginnt die Pumpe zu fordern, bis der obere Grenzdruck wieder erreicht ist.

Die Position des Kolbens des Druckumsetzers ist mittels eines diesem zugeordneten Wegmesssensors, vorzugsweise einem Potentiometer, erfassbar. Diese Wegerfassung ist wichtig für die Regelung sowie Überwachung der jeweiligen Injektionsparameter.

Um zu verhindern, dass der Kolben eines Druckumsetzers in dessen Stillstandszeit durch äußere Kräfte unbeabsichtigt verschoben wird, sollte der Kolben dieses Druckumsetzers zu gleicher Zeit derart von beiden Seiten mit Druck beaufschlagbar sein, dass der Kolben in Ruhe verharrt. Bei einem solchen Druckregelsystem wird unabhängig von evtl. äußeren auf den Kolben einwirkenden Kräften stets die Einhaltung der gerade gewünschten Verharrungsposition sichergestellt.

Bei medizintechnischen Systemen mit hydraulischen Komponenten stellt insbesondere der Aspekt der Entlüftung des Hydrauliksystems einen nicht unerheblichen Problempunkt dar. Um hier Abhilfe zu schaffen, wird erfindungsgemäß vorgeschlagen, dass die den Arbeitsräumen jeder Kolben-Zylinder-Einheit zugeordneten Flächen des Kolbens unterschiedlich groß sind und ein Bypassquerschnitt, vorzugsweise innerhalb des Kolbens, vorhanden ist, durch den bei einer Druckdifferenz zwischen den Arbeitsräumen stets ein geringer Leckagestrom fließt. Nach der Erfindung wird somit bewusst ein geringer Leckagestrom durch die Kolben-Zylinder-Einheiten geschaffen, um eine fortlaufende Umwälzung der Hydraulikflüssigkeit zu erreichen. Es versteht sich, dass der Bypassquerschnitt lediglich so groß bemessen sein darf, dass ein hinreichender Druckaufbau über dem Kolben weiterhin möglich ist, um die erforderlichen Verschiebekräfte zu erzeugen. Der Leckagestrom beträgt daher maximal wenige Prozent des Volumenstroms, der bei der Verschiebung des Kolbens mit maximaler Geschwindigkeit zur Verfügung gestellt wird.

Durch die ständige Umwälzung der Hydraulikflüssigkeit, d.h. die Kreislaufführung, gelangt die Flüssigkeit auch in den Hydrauliktank. Nach der Erfindung wird weiter vorgeschlagen, dass eine Oberfläche der Hydraulikflüssigkeit in dem Hydraulikbehälter mit einem Unterdruck, vorzugsweise einem Vakuum, beaufschlagt ist, welcher mittels einer Unterdruckpumpe erzeugt und/oder fortlaufend aufrechterhalten wird. Die in den Hydraulikbehälter gelangenden Luft- bzw. Gasblasen werden auf diese Weise von dem Unterdruck absorbiert bzw. von der Unterdruckpumpe aus dem System befördert.

Neben dieser kontinuierlich ablaufenden Entlüftungsfunktion ist es bisweilen wünschenswert, auch eine rasche Entfernung etwaiger Gasblasen mit Hilfe eines großen, im Kreislauf geführten Volumenstroms zu erreichen. Zu diesem Zweck ist vorgesehen, dass in einer Entlüftungsstellung des Kolbens der Kolben-Zylinder-Einheit die vorzugsweise eine Totpunktstellung ist, ein gegenüber dem Bypassquerschnitt vergrößerter Entlüftungs-Bypassquerschnitt vorhanden ist, wobei der Entlüftungs-Bypassquerschnitt vorzugsweise von einer Querschnittserweiterung des Zylinders der Kolben-Zylinder-Einheit gebildet ist. Solange sich der Kolben somit in der Entlüftungsstellung befindet, schließt ein großer Volumenstrom im Kreislauf und führt auf diese Weise zu einer schnellen Entfernung der Gaseinschlüsse aus dem System und einem Abtransport in den Hydraulikbehälter. Für den Betrieb der Kolben-Zylinder-Einheit ist der große Entlüftungs-Bypassquerschnitt unkritisch, da während der Entlüftung keine Kräfte über den Abtrieb aufgebracht werden müssen. Der Entlüftungs-Bypassquerschnitt muss jedoch so begrenzt werden, dass bei einer Freiheit des Abtriebs an äußeren Kräften zumindest die Entlüftungsstellung wieder verlassen werden kann, um den Entlüftungs-Bypassquerschnitt automatisch wieder zu verschließen, so dass dann wieder entsprechend große Kräfte über hinreichende Druckdifferenzen über den Kolben erzeugt werden können.

Gemäß einer Ausgestaltung der Erfindung ist vorgeschlagen, dass die Querschnittserweiterung des Zylinders in der Entlüftungsstellung in Form einer in der inneren Mantelfläche des Zylinders befindlichen, die axiale Länge des Kolbens überbrückenden Ausnehmung und/oder Kanals ausgeführt ist.

Ein Verfahren zur Injektion von Flüssigkeit in einen Patienten während einer Magnet-Resonanz-Tomografie durch ein MRT-System, wobei durch Verlagerung eines Förderelements mit Hilfe einer Antriebseinrichtung Flüssigkeit in einen Patienten injiziert wird; ist erfindungsgemäß ausgestaltet durch eine hydraulische Antriebseinrichtung deren Druckbeaufschlagung über Hydraulikleitungen von innerhalb öder außerhalb des Raums her erfolgt.

Um eine unbeabsichtigte Verschiebung des Kolbens eines Druckumsetzers während dessen Stillstandszeiten zu vermeiden, ist vorgesehen, dass der Kolben in diesen Zeiten auf die jeweilige Stillstandsposition eingeregelt wird, um ihn somit unempfindlich gegen Verschiebungen durch äußere Kräfte zu machen. Dabei wird als Regelgröße der Kolbenweg herangezogen und über einen Wegsensor erfasst Als Stellgrößen werden vorteilhafter Weise die in den beiden Arbeitsräumen wirkenden Drücke herangezogen, wobei beide Drücke stets oberhalb eines bestimmten Mindestdrucks gehalten werden.

Im übrigen sollten auch die Drücke in sämtlichen Hydraulikleitungen des erfindungsgemä-Ben Systems auch in Stillstandszeiten des jeweiligen Systemteils oberhalb eines bestimmten Mindestdrucks gehalten werden, um eventuelle Undichtigkeiten rasch erkennen zu können.

Dabei ist erfindungsgemäß eine Hydraulikleitung auch als Steuerleitung zur Ansteuerung bestimmter Funktionen des Injektionsvorgangs verwendbar. Diese ist vorteilhaft mit einer Start-Funktion zur Abgabe der Flüssigkeit an den Patienten, einer Stop-Funktion zum Anhalten des Injektionssystems, einer Befüll-Funktion zum Aufziehen von Flüssigkeit durch das Förderelement und/oder einer Entlüftungsfunktion zum Entlüften des Förderelements auszustatten, indem etwa durch mechanische Signalgeber Signale erzeugt werden, um außerhalb des Raums entsprechend der Funktion umgesetzt zu werden.

Um eine einfache und zuverlässige Entlüftung des verwendeten Hydrauliksystems zu ermöglichen, wird erfindungsgemäß vorgeschlagen, dass andauernd ein gewisser geringer Volumenstrom durch einen Bypassquerschnitt durch die Kolben-Zylinder-Einheit aufrechterhalten wird, der zu dem Hydraulikbehälter zurückgeführt wird, in dem eine Oberfläche der Hydraulikflüssigkeit, vorzugsweise mittels einer Unterdruckpumpe, mit einem Unterdruck beaufschlagt wird. Um eine besonders rasche Entlüftung möglich zu machen, ist vorgesehen, dass der Volumenstrom in einer bestimmten Stellung des Kolbens der Kolbenzylindereinheit vergrößert ist, so dass temporär eine schnellere Umwälzung der Hydraulikflüssigkeit und eine schnellere Abfuhr der eventuellen Gaseinschlüsse erfolgt.

Im Folgenden soll die Erfindung anhand eines Ausführungsbeispiels näher erläutert werden.

Es zeigt
- Fig. 1:: eine schematische Ansicht eines Injektionssystems in Verbindung mit einem MRT-System und
- Fig. 2:: eine schematische Ansicht einer Ausführungsform eines Injektionssystems in Verbindung mit einem MRT-System.

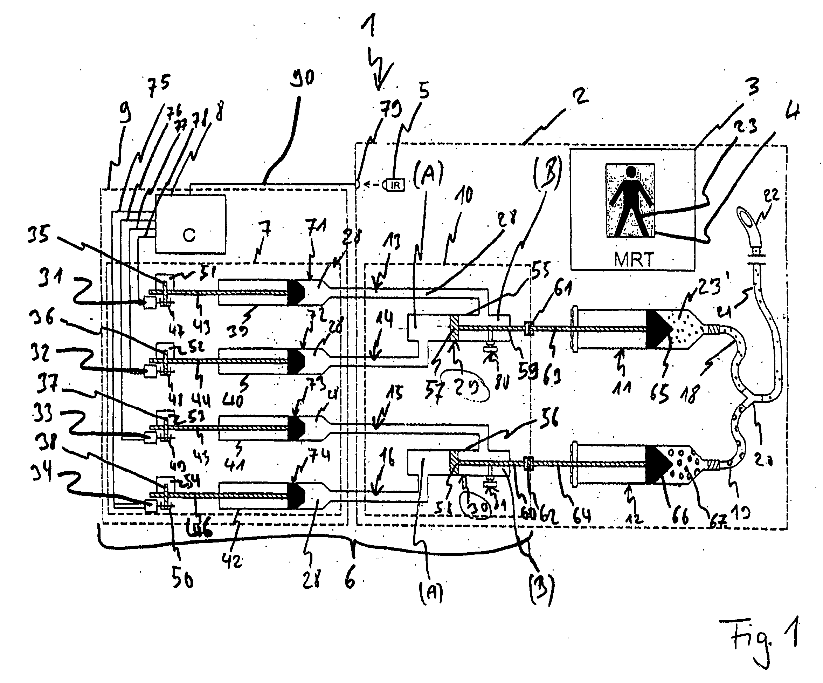

Ein in Fig. 1 gezeigte Injektionssystem 1 wird jetzt zur Erleichterung des Verständnisses der Erfindung beschrieben. Das Injektionssystem 1 ist innerhalb und außerhalb eines gegen elektromagnetische Felder abgeschirmten Raums 2 angeordnet. In dem Raum 2 befindet sich außerdem das MRT-Gerät 3 in Form einer Röhre, in welche eine Patientenliege 4 ein- und ausfahrbar gelagert ist. Das Injektionssystem 1 beinhaltet eine sich innerhalb des Raumes 2 befindliche Fernbedienung 5 von der aus Steuersignale zur Steuerung einer Injektionsvorrichtung 6 ausgegeben werden können. Die Injektionsvorrichtung 6 weist außerdem vier außerhalb des Raumes 2 angeordnete und zu einer Druckerzeugungseinrichtung 7 zusammengefasste Druckerzeugungseinheiten 71, 72, 73, 74 und eine Steuer- und Kontrolleinheit 8 auf, wobei diese Einheiten innerhalb eines Gehäuses 9 zusammengefasst und miteinander verbunden sind. Innerhalb des Raumes 2 weist die Injektionsvorrichtung 6 eine Antriebseinrichtung 10 und zwei daran angeschlossene Förderelemente 11, 12 in Form von Zylinder-Kolben-Einheiten; insbesondere Spritzen auf. Ebenfalls zur Injektionsvorrichtung gehören die Hydraulikleitungen 13, 14, 15, 16 welche die Antriebseinrichtung 10 und die Druckerzeugungseinheiten 71, 72, 73, 74 durch die Abschirmung des Raumes 2 hinweg verbinden. Die Antriebseinrichtung 10, die Druckerzeugungseinrichtung 7, die Steuer- und Kontrolleinheit 8 und die Hydraulikleitungen 13, 14, 15, 16 bilden die Injektionsvorrichtung 6.

Die beiden Förderelemente 11, 12 sind über Schläuche 18, 19 an ein Y-Element 20 angeschlossen. Durch eine weitere Schlauchverbindung 21 kann das Y-Element 20 mit einer Injektionsnadel 22 verbunden werden, die einem Patienten 23 zur Gabe von Kontrastmittel 23' während einer MRT-Untersuchung gelegt wird.

Im folgenden sollen die aufgezählten Geräte und Elemente näher erläutert werden.

Die Druckerzeugungseinheiten 71, 72, 73, 74 fördern Hydraulikflüssigkeit 28 in und aus der im Raum 2 befindlichen Antriebseinrichtung 10. Dabei sind jeweils zwei Druckerzeugungseinheiten durch je eine Hydraulikleitung 13, 14, 15, 16 mit zwei Druckumsetzern 29, 30 der Antriebseinrichtung 10 verbunden. Die Druckerzeugungseinheiten 71, 72, 73, 74 der Druckerzeugungseinrichtung 7 bestehen jeweils aus einem Antrieb 31, 32, 33, 34 in Form eines E-Motors, welcher eine Spindel 35, 36, 37, 38 antreibt, sowie einer Zylinder-Kolben-Einheit 39, 40, 41, 42 an der endseitig jeweils eine Hydraulikleitung 13, 14, 15, 16 angeschlossen ist. Die Zylinder-Kolben-Einheit 39, 40, 41, 42 besteht aus dem Zylinder und einem darin linear verschiebbaren Kolben, welcher mit einer ein Gewinde aufweisenden Kolbenstange 43, 44, 45, 46 verbunden ist. Die Kolbenstange 43, 44, 45, 46 -und damit der Kolben- ist im übrigen gegen ein Verdrehen dadurch geschützt, dass eine hier nicht gezeigte Feder des Zylinders in eine ebenfalls nicht gezeigte Nut der Kolbenstange 43, 44, 45, 46 eingreift. Der Antrieb 31, 32, 33, 34 und die Zylinder-Kolben-Einheit 39, 40, 41, 42 sind dadurch verbunden, dass die Spindel 35, 36, 37, 38 mittels eines Innengewindes auf die Kolbenstange 43, 44, 45, 46 aufgeschraubt ist. Durch eine äußere Verzahnung der Spindel 35, 36, 37, 38 kann der E-Motor mittels eines mit seiner Welle verbundenen Zahnrades 47, 48, 49, 50 in die Spindel 35, 36, 37, 38 eingreifen und diese drehen. Durch die Drehung der Spindel 35, 36, 37, 38 erfährt die Kolbenstange 43, 44, 45, 46 und damit der Kolben eine laterale Bewegung im Zylinder. Durch ein Halteelement 51, 52, 53, 54 lässt sich die Spindel 35, 36, 37, 38 im übrigen nicht in lateraler Richtung bewegen. Die Drehachse der Spindel 35, 36, 37, 38 , des Zahnrades 47, 48, 49, 50 und der Welle weisen im übrigen in dieselbe laterale Richtung wie die Verschiebungsrichtung der Kolbenstange 43, 44, 45, 46.

Die Antriebseinrichtung 10 besteht aus zwei Druckumsetzern 29, 30. Ein Druckumsetzer 29, 30 besteht aus einem Zylinder-Kolben-Element 55, 56 mit einem lateral beweglichen Kolben 57, 58 sowie einer mit diesem verbundenen und nach außen geführten Abtriebselement 59, 60 in Form einer Kolbenstange. Zwischen dem Kolben 57, 58 und den Stirnseiten des Zylinder-Kolben-Elements 55, 56 befinden sich zwei durch den Kolben getrennte Arbeitsräume A, B, die durch Hydraulikanschlüsse mit den Hydraulikleitungen 13, 14, 15, 16 verbunden sind. In jeweils beiden Kammern A, B, in den Hydraulikleitungen 13, 14, 15, 16 sowie in den Zylinderkammern der Druckerzeugungseinheiten 71, 72, 73, 73 befindet sich Hydraulikflüssigkeit 28, die unter einem Druck von etwa 0,5 bis 2 bar steht.

Am freien Ende der Kolbenstange greift ein Rastelement 61, 62 ein. Das Rastelement ist mit einer Kolbenstange 63, 64 der Fördereinheit 11, 12 in Form einer Zylinder-Kolben-Einheit, insbesondere einer Spritze verbunden. Mit der Kolbenstange 63, 64 ist ein Kolben 65, 66 verbunden der innerhalb der Spritze verschiebbar ist. Eine Spritze dient der Aufnahme und Abgabe von Kochsalzlösung 67, während die andere Spritze das Kontrastmittel 23' aufnimmt bzw. wieder abgibt.

Die Elektromotoren der Druckerzeugungseinheit 7 werden von der Steuer- und Kontrolleinheit 8 über Verbindungsleitungen 75, 76, 77, 78 mit Strom versorgt. Auch werden der Steuer- und Kontrolleinheit 8 Sensordaten hier nicht gezeigter Sensoren zugeführt. Die Sensoren messen den Druck in allen Hydraulikleitungen 13, 14, 15, 16 die Position der Kolbenstangen 43, 44, 45, 46 der Zylinder-Kolben-Einheiten 39, 40, 41, 42, die Temperatur der Motoren, die Temperatur der Hydraulikflüssigkeit 28 und/oder die Dehnung von Abschnitten der Hydraulikleitungen 13, 14, 15, 16 . Somit sind die Sensoren vor allem der Druckerzeugungseinrichtung 7 zugeordnet und innerhalb dieser installiert. Es ist nicht erforderlich, die Sensoren innerhalb des Raumes 2 zu installieren, da alle genannten Parameter auch außerhalb des Raumes ermittelt werden können.

Über geeignete Auswertesoftware der Steuer- und Kontrolleinheit 8 wird das Injektionssystem 1 betriebsbereit gehalten, d.h. im wesentlichen die Motorströme der Elektromotoren in den erforderlichen Bereichen gehalten. Dabei kann jederzeit von einer Bedienperson außerhalb des Raumes 2 durch geeignete Eingabemittel, wie eine hier nicht gezeigte PC-Tastatur in den Kontroll- und Steuerungsablauf des Injektionssystems 1 eingegriffen werden. Ebenfalls ist es möglich, dass eine sich innerhalb des Raums 2 befindliche weitere Bedienperson das Injektionssystem 1 über die Steuer- und Kontrolleinheit 8 mittels der Fernbedienung 5 steuert. Das Infrarot-Signal welches von der Fernbedienung 5 ausgesendet wird, kann mittels einer im Raum angeordneten Empfangseinheit 79 in Form eines Infrarotempfängers empfangen und der ortsüblichen Netzspannung aufmoduliert werden. Die Empfangseinheit 79 steckt innerhalb des Raums 2 in einer Netzsteckdose. Die Netzspannung, die zum Betrieb der Steuer- und Kontrolleinheit 8 ohnehin erforderlich ist und die dieser über eine Versorgungsleitung 90 zugeführt wird, kann von der Steuer- und Kontrolleinheit 8 dekodiert werden, so dass die Signale der Fernbedienung 5 letztlich von der Software der Steuer- und Kontrolleinheit 8 weiterverarbeitet werden können.

Die zweite Bedienperson hat innerhalb des Raums zudem die Möglichkeit in den Ablauf des Injektionsvorgangs mechanisch direkt einzugreifen, indem sie an den Druckumsetzer 29, 30 vorgesehene mechanische Verriegelung 80, 81 in Form eines Rastelementes betätigt. Das Rastelement greift bei Betätigung insbesondere in das jeweilige Abtriebselement 59, 60 in Form der Kolbenstange, die zu diesem Zweck abschnittsweise mit einer Zahnung versehen ist ein und verhindert die weitere Abgabe von Kontrastmittel 23' bzw. Kochsalzlösung 67 durch die Injektionsvorrichtung 6.

Die Abgabe bzw. Aufnahme von Flüssigkeit durch die Förderelemente 11, 12 wird ausschließlich durch das Druckverhältnis zwischen den beiden Arbeitsräume A, B der Druckerzeugungseinheiten 29, 30 bestimmt.

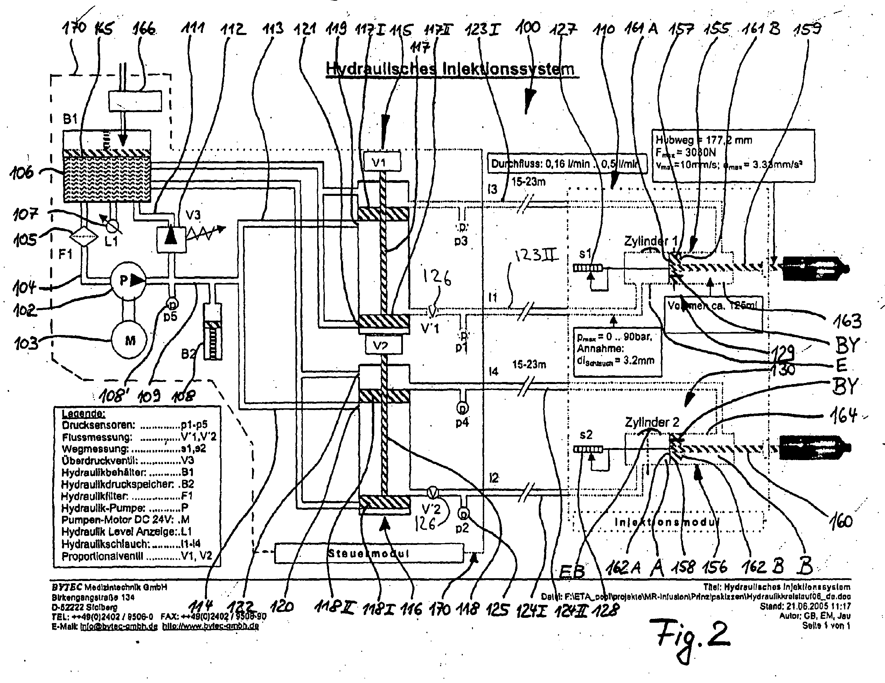

Fig. 2 zeigt ein alternatives Injektionssystem 100, bei dem anstelle von Kolben-Zylinder-Einheiten für die Druckerzeugung für das gesamte System eine einzige Hydraulikpumpe 102 vorgesehen ist, die von einem Motor 103 angetrieben ist. Vom Prinzip her unverändert ist gegenüber dem in Fig. 1 gezeigten System die Antriebseinrichtung 110, die wiederum aus zwei Zylinder-Kolben-Einheiten 155, 156 bestehen. In Fig. 2 nicht gezeigt, jedoch auch bei dieser Variante des Injektionssystems 100 vorhanden, sind die beiden Förderelemente in Form von Spritzen zur Injektion eines Kontrastmittels einerseits und einer Kochsalzlösung andererseits. Nicht dargestellt sind auch die Patientenliege sowie der Patient selbst, der sich beispielsweise innerhalb des röhrenförmigen Magneten des MRT-Systems befindet.

Die Hydraulikpumpe 102 saugt über eine Saugleitung 104 Hydraulikflüssigkeit über ein Hydraulikfilter 105 aus einem Hydraulikbehälter 106 in dem die Hydraulikflüssigkeit unter einem Überdruck gegenüber dem Atmosphärendruck steht. Ein Füllstandsmesser 107 erfasst das Niveau der Hydraulikflüssigkeit in dem Hydraulikbehälter 106.

Auf der Druckseite der Hydraulikpumpe 102 befindet sich zum einen ein Druckspeicher 108, der dafür sorgt, dass auch bei Stillstand der Hydraulikpumpe 102 stets ein gewisses Volumen unter Druck stehender Hydraulikflüssigkeit auf der Druckseite entnommen werden kann. Ein Drucksensor 108' erfasst stets den Druck und sorgt dafür, das die nicht dargestellte Steuer- und Kontrolleinheit ein Einschaltsignal bei Unterschreitung eines unteren Grenzdrucks und ein Abschaltsignal bei Überschreiten eines oberen Grenzdrucks erhält. Zum anderen geht von der Druckleitung 109 auch eine Leitung 111 ab, in der sich ein überstellbares Überdruckventil 112 befindet und die in den Hydraulikbehälter 106 zurückführt.

Die Druckleitung 109 teilt sich in zwei Leitungen 113 und 114 auf, die jeweils zu einem Proportionalventil 115, 116 führen, mit welchen die Zumessung der Hydraulikflüssigkeit zu den beiden Arbeitsräumen A, B der beiden Kolben-Zylinder-Einheiten 155 und 156 übernimmt.

Die beiden Proportionalventile 115, 116 besitzen jeweils eine Kolbenstange 117, 118, an denen sich jeweils im Abstand zueinander zwei Kolben 117 1, 117 11 und 118 1, 118 11 finden. Jedes der Proportionalventile 115, 116 ist mit einem mittigen Anschluss 119, 120 für die unter Druck stehende Hydraulikflüssigkeit versehen, sowie zwei in der Nähe der Stirnseiten angeordneten Anschlüssen 121, 122 die jeweils über Leitungen mit dem Hydraulikbehälter 106 verbunden sind.

Von der gegenüberliegenden Seite der Proportionalventile 115, 116 gehen jeweils zwei Leitungen 123 1, 123 11 sowie 124 1,124 II aus, die zu den Arbeitsräumen A bzw. B der Kolben-Zylinder-Einheiten 155, 156 führen. In jeder dieser Leitungen 123 I, 123 11 und 124 1, 124 11, befindet sich jeweils ein Drucksensor 125 und in den Leitungen 123 II und 124 1 ein Volumenstrommesser 126.

Je nach Stellung der Kolbenstangen 117, 118 bzw. der damit verbundenen Kolben 117 I, 117 II bzw. 118 1, 118 II wird ein mehr oder weniger großer Volumenstrom in Richtung eines Arbeitsraumes A oder B der Zylinderkolbeneinheiten 155, 156 und ein entsprechend großer Rückfluss aus dem jeweils anderen Arbeitsraum B oder A in das Proportionalventil 115, 116 freigegeben.

Die jeweilige Stellung des Kolbens 157, 158 der Kolben-Zylinder-Einheiten 155, 156 bzw. der damit verbundenen Kolbenstangen in Form der Abtriebselemente 159, 160 (die zu den nicht gezeigten Förderelementen in Form von Spritzen führen) ist mit Hilfe der Wegsensoren 127, 128 erfassbar. Mit Hilfe eines Regelalgorithmus, der in die bildlich nicht näher dargestellte Steuer- und Kontrolleinrichtung integriert ist, wird die Position der Kolben 157, 158 zu Zeiten, in denen diese eine Verharrungsposition einnehmen sollen, dadurch unveränderlich gehalten, dass bei Einwirkung äußerer Kräfte über die Abtriebselemente 159, 160 die Drücke in den Arbeitsräumen A und B so eingeregelt werden, dass keine Lageveränderung der Kolben 157, 158 eintritt. Die Proportionalventile 115, 116 werden von der Steuer- und Kontrolleinrichtung entsprechend angesteuert.

Eine Erstfehlersicherheit des Injektionssystems 100 besteht insofern, als mit Hilfe der Volumenstrommesser 126 und der Drucksensoren 125 überprüft werden kann, ob bei einer gemessenen Verlagerung der Kolben 157, 158 zu erwartenden Druck- und Volumenstromverhältnisse in den jeweiligen Leitungen 123 I, 123 II bzw. 124 I, 124 II auch tatsächlich vorliegen.

Bei dem in Fig. 2 gezeigten Injektionssystem 100 kann die Druckerzeugungseinrichtung 170 sowohl innerhalb als auch außerhalb des Raumes angeordnet sein, in dem sich die große Feldstärke aufweisende Einrichtung, d.h. bei MRT-System insbesondere der Magnet, befindet. Sofern Druckerzeugungseinheit 170 sich innerhalb des Raumes befindet, ist sie vorzugsweise soweit wie möglich bzw. erforderlich von dem Magneten selbst entfernt positioniert, so dass im Bereich mit der größten Feldstärke lediglich noch die Hydraulikleitungen 123 I, 123 II und 124 I, 124 II verlaufen und insbesondere keine elektrischen Leitungen mehr, mit denen elektrische Leistungskomponenten betrieben werden müssten, wie beispielsweise Stellmotoren für die mechanische Betätigung der Förderelemente.

Um eine einfache und zuverlässige Entlüftung des Hydrauliksystems zu bewerkstelligen, sind die Flächen 161A, 161B, 162A, 162B der Kolben 157, 158 der Kolben-Zylinder-Einheiten 155, 156 unterschiedlich groß ausgeführt. Dies wird erreicht durch eine unterschiedliche Querschnittsfläche der Kolbenstange 159 im Bereich der beiden Arbeitsräume A, B. Während die Kolbenstange 159 auf der Abtriebsseite, d.h. im Bereich des Arbeitsraums B, einen größeren Querschnitt aufweist, ist sie im Bereich des Arbeitsraums A mit einem geringeren Durchmesser ausgeführt, da hier lediglich der Auslesevorgang des Wegsensors 127, 128 erfolgen muss.

Aufgrund der unterschiedlichen Flächen 161A zu 161B bzw. 162A zu 162B muss auch im Verharrungszustand des Kolbens 157, 158 stets eine gewisse Druckdifferenz zwischen den beiden Arbeitsräumen A und B bestehen. Es ist nun in den Kolben 157, 158 jeweils ein Bypassquerschnitt B in Form einer kleinen Bohrung vorhanden. Durch den Bypassquerschnitt B liegt stets ein geringer Volumenstrom vor, der eine Kreislaufführung der Hydraulikflüssigkeit bewirkt. Eventuell im System vorhandene Gasblasen werden somit über die entsprechenden Hydraulikleitungen bis zu dem Hydraulikbehälter 106 geführt.

Die Oberfläche 165 der Hydraulikflüssigkeit ist mit Hilfe einer Unterdruckpumpe 166 mit einem Unterdruck beaufschlagt, so dass Gasblasen die Hydraulikflüssigkeit verlassen und mittels der Unterdruckpumpe 166 aus dem System abgeführt werden.

Um bedarfsweise auch eine rasche Entlüftung zu ermöglichen, ist in einer Entlüftungsstellung E des Kolbens 155, 156 ein im inneren Mantel des Zylinders 163, 164 angeordneter Entlüftungs-Bypassquerschnitt EB vorhanden. Dieser bewirkt in der Entlüftungsstellung E, die der linken Totpunktposition der Kolben 157, 158 entspricht, eine Entlüftung über einen gegenüber dem Bypassquerschnitt B in den Kolben 157, 158 deutlich vergrößerten Volumenstrom. Die Abfuhr der evtl. Gasblasen erfolgt auch im Falle dieser "Schnellentlüftung" über die Unterdruckpumpe 166, die an den Hydraulikbehälter 106 angeschlossen ist.

### Bezugszeichenliste

- 1: Injektionssystem
- 2: Raum
- 3: MRT-Gerät
- 4: Patientenliege
- 5: Fernbedienung
- 6: Injektionsvorrichtung
- 7: Druckerzeugungsvorrichtung
- 8: Steuer- und Kontrolleinheit
- 9: Gehäuse
- 10: Antriebseinrichtung
- 11, 12: Förderelement
- 13, 14, 15, 16: Hydraulikleitung
- 18, 19: Schlauch
- 20: Y-Element
- 21: Schlauchverbindung
- 22: Injektionsnadel
- 23: Patient
- 23': Kontrastmittel
- 28: Hydraulikflüssigkeit
- 29,30: Druckumsetzer
- 31, 32, 33, 34: Antrieb
- 35, 36, 37, 38: Spindel
- 39, 40, 41, 42: Zylinder-Kolben-Einheit
- 43, 44, 45, 46: Kolbenstange
- 47, 48, 49, 50: Zahnrad
- 51, 52, 53, 54: Halteelement
- 55, 56: Zylinder-Kolben-Element
- 57,58: Kolben
- 59,60: Abtriebselement
- 61, 62: Rastelement
- 63, 64: Kolbenstange
- 65, 66: Kolben
- 67: Kochsalzlösung
- 71, 72, 73, 74: Druckerzeugungseinheit
- 75, 76, 77, 78: Verbindungsleitungen
- 79: Empfangseinheit
- 80, 81: Verriegelung
- 90: Versorgungsleitung
- 100: Injektionssystem
- 102: Hydraulikpumpe
- 103: Motor
- 104: Saugleitung
- 105: Filter
- 106: Hydraulikbehälter
- 107: Füllstandsmesser
- 108: Druckspeicher
- 108': Drucksensor
- 109: Druckleitung
- 110: Antriebseinrichtung
- 111: Leitung
- 112: Überdruckventil
- 113, 114: Leitung
- 115, 116: Proportionalventil
- 117, 118: Kolbenstange
- 117I/II, 118I/II: Kolben
- 119, 120: Anschluss
- 121, 122: Anschluss
- 123I/II, 124I/II: Leitung
- 125: Drucksensor
- 126: Volumenstrommesser
- 127, 128: Wegsensoren
- 129, 130: Druckumsetzer
- 155, 156: Kolben-Zylinder-Einheit
- 157, 158: Kolben
- 159, 160: Antriebselement
- 161A/B, 162 A/B: Flächen
- 163, 164: Zylinder
- 165: Oberfläche
- 166: Unterdruckpumpe
- B: Bypassquerschnitt
- E: Entlüftungsstellung
- EB: Entlüftungs-Bypassquerschnitt
- 170: Druckerzeugungseinrichtung

## Patentansprüche

1. Injektionssystem (100) zur Injektion von Flüssigkeiten innerhalb eines starken Magnetfelds, eines magnetischen Wechselfelds und/oder eines elektrischen Hochfrequenzfelds zur Verwendung mit einem medizinisch-technischen System mit
a) einem gegen elektromagnetische Felder mittels einer Abschirmung abgeschirmten Raum,
b) einer Injektionsvorrichtung, durch die zu injizierende Flüssigkeit in einen Patienten abgebbar ist,
c) einer Antriebseinrichtung (110) der Injektionsvorrichtung, mit der mindestens ein Förderelement der Injektionsvorrichtung zur Injektion verlagerbar ist, wobei die Antriebseinrichtung einen Druckumsetzer (129, 130) mit einem bewegbaren Kolben (157, 158) aufweist,
d) einer außerhalb des Raumes angeordneten Steuer- und Kontrolleinheit,
e) einer hydraulischen Antriebseinrichtung (110), deren mindestens eine Hydraulikleitung (123 I, 123 II, 124 I, 124 II) aus dem Raum heraus zu einer Druckerzeugungseinheit (170) in Form einer Hydraulikpumpe (102) geführt ist,
**gekennzeichnet durch** einen in dem abgeschirmten Raum angeordneten Wegmesssensor (127, 128), vorzugsweise ein Potentiometer, mit dem die Position des Kolbens (157, 158) des zugeordneten Druckumsetzers (129, 130) erfassbar ist.

2. Injektionssystem (100) nach Anspruch 1, **dadurch gekennzeichnet, dass** das medizinisch-technische System ein MRT-System beinhaltet.

3. Injektionssystem (100) nach Anspruch 1 oder 2, **dadurch gekennzeichnet, dass** das medizinisch-technische System ein Computer-Tomografie-System beinhaltet.

4. Injektionssystem nach mindestens einem der Ansprüche 1 bis 3, **dadurch gekennzeichnet, dass** die Druckerzeugungseinheit oder eine Druckerzeugungseinrichtung in die Steuer- und Kontrolleinheit integriert ist.

5. Injektionssystem nach mindestens einem der Ansprüche 1 bis 4, **dadurch gekennzeichnet, dass** es innerhalb des Raumes aus im Wesentlichen magnetisch und elektrisch nicht leitfähigem Material besteht.

6. Injektionssystem nach mindestens einem der Ansprüche 1 bis 5, **dadurch gekennzeichnet, dass** die mindestens eine Hydraulikleitung abgedeckt innerhalb des Raumes mindestens zu einem Teil in und/oder auf dem Boden, der Wand und/oder der Decke verläuft.

7. Injektionssystem nach mindestens einem der Ansprüche 1 bis 6, **dadurch gekennzeichnet, dass** die mindestens eine Hydraulikleitung (123I, 123II, 124I, 124II) in oder an einem Ableitungsschacht des medizinisch-technischen Systems verläuft, wobei der Ableitungsschacht aus dem Raum herausgeführt wird.

8. Injektionssystem nach den mindestens einem der Ansprüche 1 bis 7, **dadurch gekennzeichnet, dass** die Hydraulikleitungen (123I, 123II, 124I, 124II) mit biokompatibler Hydraulikflüssigkeit befüllt sind.

9. Injektionssystem nach mindestens einem der Ansprüche 1, bis 8, **dadurch gekennzeichnet, dass** die Hydraulikflüssigkeit unter einem Ruhedruck von vorzugsweise 0,5 bis 2 bar in den Hydraulikleitungen (123I, 123II, 124I, 124II) vorliegt.

10. Injektionssystem (100) nach mindestens einem der Ansprüche 1 bis 9, **dadurch gekennzeichnet, dass** die hydraulische Antriebseinrichtung (110) einen innerhalb des Raumes angeordneten Druckumsetzer (129, 130) aufweist, dessen Abtriebselement (159, 160)mit dem Förderelement gekoppelt ist.

11. Injektionssystem nach mindestens einem der Ansprüche 1 bis 10 **dadurch gekennzeichnet, dass** der Druckumsetzer (129, 130) eine Kolben-Zylinder-Einheit mit zwei Arbeitsräumen (A, B) aufweist, die durch einen an das Abtriebselement (159, 160) gekoppelten Kolben (157, 158) voneinander getrennt sind.

12. Injektionssystem nach mindestens einem der Ansprüche 1 bis 11, **dadurch gekennzeichnet, dass** die hydraulische Antriebseinrichtung am MRT-System befestigbar ist.

13. Injektionssystem nach mindestens einem der Ansprüche 1 bis 12, **dadurch gekennzeichnet, dass** die Antriebseinrichtung durch eine mechanische Verriegelung verriegelbar ist.

14. Injektionssystem nach mindestens einem der Ansprüche 1 bis 13, **dadurch gekennzeichnet, dass** die Druckerzeugungseinheit durch ein Steuersignal einer Fernbedienung, die sich im Raum befindet, steuerbar ist.

15. Injektionssystem nach Anspruch 14, **dadurch gekennzeichnet, dass** die Fernbedienung Steuersignale unidirektional aussendet und eine Empfangseinheit innerhalb des Raumes die Steuersignale empfängt und über eine Versorgungsleitung in das elektrische Netz einspeist, von wo aus die Steuersignale außerhalb des Raumes auslesbar sind.

16. Injektionssystem nach mindestens einem der Ansprüche 14 und 15, **dadurch gekennzeichnet, dass** die Steuersignale Infrarot-Signale sind und durch ein Fenster des Raums oder durch Modulation der Steuersignale auf eine Netzfrequenz durch die Empfangseinheit, welche in eine an die Versorgungsleitung angeschlossene Steckdose einsteckbar ist, übertragbar sind.

17. Injektionssystem (100) nach mindestens einem der vorstehenden Ansprüche, **dadurch gekennzeichnet, dass** der mindestens eine Druckumsetzer (129, 130) jeweils über ein Proportionalventil (115, 116) mit der Druckseite der Hydraulikpumpe (102) beaufschlagbar ist.

18. Injektionssystem (100) nach mindestens einem der vorstehenden Ansprüche, **dadurch gekennzeichnet, dass** das Proportionalventil (115, 116) mit mindestens einem der beiden Arbeitsräume (A, B) des zugeordneten Druckumsetzers (129, 130) verbunden ist.

19. Injektionssystem (100) nach mindestens einem der vorhergehenden Ansprüche, **gekennzeichnet durch** einen auf der Druckseite der Hydraulikpumpe (102) angeordneten Druckspeicher (108) sowie einem Drucksensor (108'), mit dem der Druck auf der Druckseite der Hydraulikpumpe (102) messbar und die Hydraulikpumpe (102) bei Überschreiten eines oberen Grenzdrucks abschaltbar und bei Unterschreiten eines unteren Grenzdrucks einschaltbar ist.

20. Injektionssystem (100) nach mindestens einem der vorhergehenden Ansprüche, **dadurch gekennzeichnet, dass** der Kolben (157, 158) jedes Druckumsetzers (129, 130) zu gleicher Zeit derartig von beiden Seiten mit Druck beaufschlagbar ist, dass der Kolben (157, 158) in Ruhe verharrt.

21. Injektionssystem (100) nach mindestens einem der vorstehenden Ansprüche, **dadurch gekennzeichnet, dass** die den Arbeitsräumen (A,B) jeder Kolben-Zylinder-Einheit (155, 156) zugeordneten Flächen (161, 162) des Kolbens (157, 158) unterschiedlich groß sind und ein Bypassquerschnitt (B), vorzugsweise innerhalb des Kolbens (157, 158), vorhanden ist, durch den bei einer Druckdifferenz zwischen den Arbeitsräumen (A, B) stets ein geringer Leckagevolumenstrom fließt.

22. Injektionssystem (100) nach mindestens einem der vorstehenden Ansprüche, **dadurch gekennzeichnet; dass** in einer Entlüftungsstellung des Kolbens (157, 158) der Kolben-Zylinder-Einheit (155, 156), die vorzugsweise eine Totpunktstellung des Kolbens 157, 158 ist, ein gegenüber dem Bypassquerschnitt (B) vergrößerter Entlüfturigs-Bypassquerschnitt (EB) vorhanden ist, wobei der Entlüftungs-Bypassquerschnitt (EB) vorzugsweise von einer Querschnittserweiterung des Zylinders (163, 164) der Kolben-Zylinder-Einheit (155, 156) gebildet ist.

23. Injektionssystem (100) nach mindestens einem der vorstehenden Ansprüche, **dadurch gekennzeichnet, dass** die Querschnittserweiterung in Form einer in der inneren Mantelfläche des Zylinders (163, 164) befindlichen die axiale Länge des Kolbens (157, 158) überbrückenden Ausnehmung und/oder Kanals ausgeführt ist.

24. Injektionssystem (100) nach mindestens einem der vorstehenden Ansprüche, **dadurch gekennzeichnet, dass** eine Oberfläche (165) der Hydraulikflüssigkeit in dem Hydraulikbehälter (106) mit einem Unterdruck, vorzugsweise einem Vakuum, beaufschlagt ist, welcher mittels einer Unterdruckpumpe (166) erzeugt und/oder fortlaufend aufrechterhalten wird.

25. Verfahren zur Regelung eines Injektionssystem (100) zur Injektion von Flüssigkeit in einen Patienten innerhalb eines starken Magnetfelds, eines magnetischen Wechselfelds und/oder eines elektrischen Hochfrequenzfelds, zur Verwendung in Verbindung mit einem medizinisch-technischen Verfahren, mit einem gegen elektromagnetische Felder mittels einer Abschirmung abgeschirmten Raum, wobei durch Verlagerung eines Förderelements mit Hilfe einer Antriebseinrichtung (110), die einen Druckumsetzer mit einem bewegbaren Kolben aufweist, Flüssigkeit zur Injektion in einen Patienten abgegeben wird, wobei eine Druckerzeugung mittels einer Hydraulikpumpe und eine Druckbeaufschlagung einer hydraulischen Antriebseinrichtung (110), über Hydraulikleitungen (123 I, 123 II, 124 I, 124 II) erfolgt, **dadurch gekennzeichnet, dass** die Position des Kolbens des Druckumsetzers mittels eines in dem abgeschirmten Raum angeordneten Wegmesssensors, vorzugsweise eines Potentiometers, erfasst wird.

26. Verfahren nach Anspruch 25, **dadurch gekennzeichnet, dass** die Druckbeaufschlagung von außerhalb des Raums erfolgt.

27. Verfahren nach Anspruch 25 oder 26, **dadurch gekennzeichnet, dass** in Zeiten, in denen der Kolben (157, 158) eines Druckumsetzers (129, 130) stillstehen soll, der Kolben (157, 158) auf die jeweilige Stillstandsposition eingeregelt wird, wobei die Regelgröße Kolbenweg über einen Wegmesser (127, 128) erfasst und als Stellgrößen die in den beiden Arbeitsräumen (A,B) wirkenden Drücke herangezogen werden, wobei beide Drücke stets oberhalb eines bestimmten Mindestdrucks gehalten werden.

28. Verfahren nach mindestens einem der Ansprüche 25 bis 27, **dadurch gekennzeichnet, dass** die Drücke in sämtlichen Hydraulikleitungen auch in Stillstandszeiten des jeweiligen Kolbens stets oberhalb eines bestimmten Mindestdruck gehalten werden.

29. Verfahren nach mindestens einem der Ansprüche 25 bis 28, **dadurch gekennzeichnet, dass** die mindestens eine Hydraulikleitung (123 I, 123 II, 124 I, 124 II) auch als Steuerleitung zur Ansteuerung mindestens einer bestimmten Funktion des Injektionsvorgangs verwendet wird.

30. Verfahren nach mindestens einem der Ansprüche 25 bis 29, **dadurch gekennzeichnet, dass** die mindestens eine Funktion
a) eine Start-Funktion zur Abgabe der Flüssigkeit,
b) eine Stop-Funktion zum Anhalten des Injektionssystems,
c) eine Befüll-Funktion zum Aufziehen von Flüssigkeit durch das Förderelement und/oder
d) eine Entlüftungsfunktion zum Entlüften des Förderelements
ist.

31. Verfahren nach mindestens einem der Ansprüche25 bis 30, **dadurch gekennzeichnet, dass** das medizinisch-technische Verfahren eine Magnet - Resonanz - Tomografie ist.

32. Verfahren nach mindestens einem der Ansprüche 25 bis 31, **dadurch gekennzeichnet, dass** andauernd ein gewisser geringer Volumenstrom durch einen Bypassquerschnitt (B) durch die Kolben-Zylinder-Einheit (155, 156) aufrechterhalten wird, der zu dem Hydraulikbehälter (106) zurückgeführt wird, in dem eine Oberfläche (165) der Hydraulikflüssigkeit, vorzugsweise mittels einer Unterdruckpumpe (166), mit einem Unterdruck beaufschlagt wird.

## Claims

1. Injection system (100) for injecting liquids within a strong magnetic field, an alternating magnetic field and/or a high-frequency electric field, for use with a medical technical system, comprising
a) a room which is shielded from electromagnetic fields by means of a shield,
b) an injection device, by means of which liquid to be injected can be dispensed to a patient,
c) a drive device (110) of the injection device, by means of which at least one conveying element of the injection device can be displaced for injection purposes, wherein the drive device comprises a pressure transducer (129, 130) with a movable piston (157, 158),
d) a control and monitoring unit (8) which is arranged outside the room,
e) a hydraulic drive device (110), the at least one hydraulic line (123 I, 123 II, 124 I, 124 II) of which is guided out of the room to a pressure generating unit (170) in the form of a hydraulic pump (102),
**characterised by** a distance sensor (127, 128), preferably a potentiometer, which is arranged in the shielded room and by means of which the position of the piston (157, 158) of the associated pressure transducer (129, 130) can be detected.

2. Injection system (100) according to claim 1, **characterised in that** the medical technical system comprises an MRI system.

3. Injection system (100) according to claim 1 or 2, **characterised in that** the medical technical system comprises a computer tomography system.

4. Injection system according to at least one of claims 1 to 3, **characterised in that** the pressure generating unit or a pressure generating device is integrated in the control and monitoring unit.

5. Injection system according to at least one of claims 1 to 4, **characterised in that**, inside the room, it consists of essentially magnetically and electrically non-conductive material.

6. Injection system according to at least one of claims 1 to 5, **characterised in that** at least part of the at least one hydraulic line runs in a concealed manner inside the room, in and/or on the floor, the wall and/or the ceiling.

7. Injection system according to at least one of claims 1 to 6, **characterised in that** the at least one hydraulic line (123I,II,124I,II)runs in or on a shaft of the medical technical system, wherein the shaft is guided out of the room.

8. Injection system according to at least one of claims 1 to 7, **characterised in that** the hydraulic lines (123I,II,124I,II)are filled with biocompatible hydraulic fluid.

9. Injection system according to at least one of claims 1 to 8, **characterised in that** the hydraulic fluid is present in the hydraulic lines (123I, II, 124I, II) under a resting pressure of preferably 0.5 to 2 bar.

10. Injection system (100) according to at least one of claims 1 to 9, **characterised in that** the hydraulic drive device (110) comprises a pressure transducer (129,130) arranged inside the room, the driven element (159,160) of which is coupled to the conveying element.

11. Injection system according to at least one of claims 1 to 10, **characterised in that** the pressure transducer (129,130) comprises a piston/cylinder unit with two working chambers (123I, II, 124I, II), (129, 130) which are separated from one another by a piston (157,158) coupled to the driven (159,160) element.

12. Injection system according to at least one of claims 1 to 11, **characterised in that** the hydraulic drive device can be fixed to the MRI system.

13. Injection system according to at least one of claims 1 to 12, **characterised in that** the drive device can be locked by means of a mechanical lock.

14. Injection system according to at least one of claims 1 to 13, **characterised in that** the pressure generating unit can be controlled by a control signal of a remote control which is located in the room.

15. Injection system according to claim 14, **characterised in that** the remote control transmits control signals in a unidirectional manner and a receiving unit inside the room receives the control signals and feeds them via a supply line into the electrical network, from where the control signals can be read outside the room.

16. Injection system according to at least one of claims 14 and 15, **characterised in that** the control signals are infrared signals and can be transmitted through a window of the room or by modulation of the control signals to a network frequency by the receiving unit, which can be inserted into a socket connected to the supply line.

17. Injection system (100) according to at least one of the preceding claims, **characterised in that** the at least one pressure transducer (129, 130) can be acted upon by the pressure side of the hydraulic pump (102) via a respective proportional valve (115, 116).

18. Injection system (100) according to at least one of the preceding claims, **characterised in that** the proportional valve (115, 116) is connected to at least one of the two working chambers (A, B) of the associated pressure transducer (129, 130).

19. Injection system (100) according to at least one of the preceding claims, **characterised by** a pressure accumulator (108) arranged on the pressure side of the hydraulic pump (102) and also a pressure sensor (108'), by means of which the pressure on the pressure side of the hydraulic pump (102) can be measured and the hydraulic pump (102) can be switched off in the event of exceeding an upper limit and switched on in the event of falling below a lower limit.

20. Injection system (100) according to at least one of the preceding claims, **characterised in that** the piston (157, 158) of each pressure transducer (129, 130) can be acted upon by pressure from both sides at the same time in such a way that the piston (157, 158) remains at rest.

21. Injection system (100) according to at least one of the preceding claims, **characterised in that** the surfaces (161, 162) of the piston (157, 158) assigned to the working chambers (A, B) of each piston/cylinder unit (155, 156) are of different size, and there is a bypass cross section (B), preferably within the piston (157, 158), through which a small leakage flow always flows in the event of a pressure difference between the working chambers (A, B).

22. Injection system (100) according to at least one of the preceding claims, **characterised in that**, in a ventilation position of the piston (157, 158) of the piston/cylinder unit (155, 156), which is preferably a dead centre position of the piston (157, 158), there is a ventilation bypass cross section (EB) which is larger than the bypass cross section (B), wherein the ventilation bypass cross section (EB) is preferably formed by a widening of the cross section of the cylinder (163, 164) of the piston/cylinder unit (155, 156).

23. Injection system (100) according to at least one of the preceding claims, **characterised in that** the widening of the cross section is designed in the form of a recess and/or channel which is located in the inner cylindrical surface of the cylinder (163, 164) and bridges over the axial length of the piston (157, 158).

24. Injection system (100) according to at least one of the preceding claims, **characterised in that** a surface (165) of the hydraulic fluid in the hydraulic container (106) is acted upon by a negative pressure, preferably a vacuum, which is generated and/or continuously maintained by means of a negative pressure pump (166).

25. Method for controlling an injection system (100) for injecting liquid into a patient within a strong magnetic field, an alternating magnetic field and/or a high-frequency electric field, for use in conjunction with a medical technical procedure, comprising a room which is shielded from electromagnetic fields by means of a shield, wherein liquid for injection into a patient is dispensed by displacing a conveying element by means of a drive device (110) which comprises a pressure transducer with a movable piston, wherein pressure is generated by means of a hydraulic pump and pressure is exerted by means of a hydraulic drive device (110), via hydraulic lines (123 I, 123 II, 124 I, 124 II), **characterised in that** the position of the piston of the pressure transducer is detected by means of a distance sensor, preferably a potentiometer, which is arranged in the shielded room.

26. Method according to claim 25, **characterised in that** the pressure is exerted from outside the room.

27. Method according to claim 25 or 26, **characterised in that**, at times when the piston (157, 158) of a pressure transducer (129, 130) is to be stationary, the piston (157, 158) is set to the respective stationary position, wherein the control parameter of piston travel is detected via a distance sensor (127, 128) and the pressures acting in the two working chambers (A, B) are used as adjusting parameters, wherein both pressures are always kept above a certain minimum pressure.

28. Method according to at least one of claims 25 to 27, **characterised in that** the pressures in all the hydraulic lines are always kept above a certain minimum pressure, even at times when the respective piston is stationary.

29. Method according to at least one of claims 25 to 28, **characterised in that** the at least one hydraulic line (123 I, 123 II, 124 I, 124 II) is also used as a control line for actuating at least one specific function of the injection process.

30. Method according to at least one of claims 25 to 29, **characterised in that** the at least one function is
a) a start function for dispensing the liquid,
b) a stop function for stopping the injection system,
c) a filling function for the uptake of liquid by the conveying element and/or
d) a ventilation function for ventilating the conveying element.

31. Method according to at least one of claims 25 to 30, **characterised in that** the medical technical procedure is magnetic resonance imaging.

32. Method according to at least one of claims 25 to 31, **characterised in that** a certain small volume flow is continuously maintained by a bypass cross section (B) through the piston/cylinder unit (155, 156), which volume flow is returned to the hydraulic container (106) in which a surface (165) of the hydraulic fluid is acted upon by a negative pressure, preferably by means of a negative pressure pump (166).

## Revendications

1. Système d'injection (100) pour l'injection de liquides à l'intérieur d'un champ magnétique intense, d'un champ magnétique alternatif et/ou d'un champ électrique à haute fréquence, à utiliser avec un système médico-technique, comprenant:
a) une chambre protégée contre des champs électromagnétiques au moyen d'un blindage,
b) un dispositif d'injection, par lequel un liquide à injecter dans un patient peut être délivré,
c) un dispositif d'entraînement (110) du dispositif d'injection, avec lequel au moins un élément de transport du dispositif d'injection peut être déplacé en vue de l'injection, dans lequel le dispositif d'entraînement comprend un convertisseur de pression (129, 130) avec un piston mobile (157, 158),
d) une unité de commande et de contrôle disposée à l'extérieur de la chambre,
e) un dispositif d'entraînement hydraulique (110), dont au moins une conduite hydraulique (123 I, 123 II, 124 I, 124 II) est guidée hors de la chambre vers une unité de production de pression (170) sous la forme d'une pompe hydraulique (102),
**caractérisé par** un détecteur de chemin parcouru (127, 128) disposé dans la chambre protégée, de préférence un potentiomètre, avec lequel on peut détecter la position du piston (157, 158) du convertisseur de pression associé (129, 130).

2. Système d'injection (100) selon la revendication 1, **caractérisé en ce que** le système médico-technique comprend un système MRT.

3. Système d'injection (100) selon la revendication 1 ou 2, **caractérisé en ce que** le système médico-technique comprend un système de tomographie assisté par ordinateur.

4. Système d'injection selon au moins l'une quelconque des revendications 1 à 3, **caractérisé en ce que** l'unité de production de pression ou un dispositif de production de pression est intégré(e) dans l'unité de commande et de contrôle.

5. Système d'injection selon au moins l'une quelconque des revendications 1 à 4, **caractérisé en ce qu'**il se compose, à l'intérieur de la chambre, d'une matière essentiellement non conductrice magnétiquement et électriquement.

6. Système d'injection selon au moins l'une quelconque des revendications 1 à 5, **caractérisé en ce que** l'au moins une conduite hydraulique est située, en étant recouverte, à l'intérieur de la chambre au moins pour une partie dans et/ou sur le fond, la paroi et/ou le plafond.

7. Système d'injection selon au moins l'une quelconque des revendications 1 à 6, **caractérisé en ce que** l'au moins une conduite hydraulique (123 I, 123 II; 124 I, 124 II) est située dans ou sur un puits d'évacuation du système médico-technique, dans lequel le puits d'évacuation est mené hors de la chambre.

8. Système d'injection selon au moins l'une quelconque des revendications 1 à 7, **caractérisé en ce que** les conduites hydrauliques (123 I, 123 II; 124 I, 124 II) sont remplies d'un liquide hydraulique biocompatible.

9. Système d'injection selon au moins l'une quelconque des revendications 1 à 8, **caractérisé en ce que** le liquide hydraulique se trouve sous une pression au repos de préférence de 0,5 à 2 bars dans les conduites hydrauliques (123 I, 123 II; 124 I, 124 II).

10. Système d'injection (100) selon au moins l'une quelconque des revendications 1 à 9, **caractérisé en ce que** le dispositif d'entraînement hydraulique (110) comprend un convertisseur de pression (129, 130) disposé à l'intérieur de la chambre, dont l'élément de sortie (159, 160) est couplé à l'élément de transport.

11. Système d'injection selon au moins l'une quelconque des revendications 1 à 10, **caractérisé en ce que** le convertisseur de pression (129, 130) comprend une unité piston-cylindre avec deux chambres de travail (A, B), qui sont séparées l'une de l'autre par un piston (157, 158) couplé à l'élément de sortie (159, 160).

12. Système d'injection selon au moins l'une quelconque des revendications 1 à 11, **caractérisé en ce que** le dispositif d'entraînement hydraulique peut être fixé au système MRT.

13. Système d'injection selon au moins l'une quelconque des revendications 1 à 12, **caractérisé en ce que** le dispositif d'entraînement peut être verrouillé au moyen d'un verrouillage mécanique.

14. Système d'injection selon au moins l'une quelconque des revendications 1 à 13, **caractérisé en ce que** l'unité de production de pression peut être commandée par un signal de commande d'une télécommande qui se trouve dans la chambre.

15. Système d'injection selon la revendication 14, **caractérisé en ce que** la télécommande émet des signaux de commande de manière unidirectionnelle et une unité de réception reçoit les signaux de commande à l'intérieur de la chambre et les injecte par une ligne d'alimentation dans le réseau électrique, d'où les signaux de commande sont lisibles à l'extérieur de la chambre.

16. Système d'injection selon au moins l'une quelconque des revendications 14 et 15, **caractérisé en ce que** les signaux de commande sont des signaux infrarouges et sont transmissibles à travers une fenêtre de la chambre ou par modulation des signaux de commande à une fréquence de réseau par l'unité de réception qui peut être connectée dans une prise femelle raccordée à la ligne d'alimentation.

17. Système d'injection (100) selon au moins l'une quelconque des revendications précédentes, **caractérisé en ce que** l'au moins un convertisseur de pression (129, 130) peut être sollicité respectivement par le côté de refoulement de la pompe hydraulique (102) au moyen d'une vanne proportionnelle (115, 116).

18. Système d'injection (100) selon au moins l'une quelconque des revendications précédentes, **caractérisé en ce que** la vanne proportionnelle (115, 116) est raccordée à au moins une des chambres de travail (A, B) du convertisseur de pression associé (129, 130).

19. Système d'injection (100) selon au moins l'une quelconque des revendications précédentes, **caractérisé par** un accumulateur de pression (108) disposé sur le côté de refoulement de la pompe hydraulique (102) et par un détecteur de pression (108'), avec lequel on peut mesurer la pression sur le côté de refoulement de la pompe hydraulique (102) et on peut déconnecter la pompe hydraulique (102) lorsque l'on dépasse une pression limite supérieure et la connecter lorsque l'on descend en dessous d'une pression limite inférieure.

20. Système d'injection (100) selon au moins l'une quelconque des revendications précédentes, **caractérisé en ce que** le piston (157, 158) de chaque convertisseur de pression (129, 130) peut être soumis au même instant à une pression sur les deux côtés, de telle manière que le piston (157, 158) reste au repos.

21. Système d'injection (100) selon au moins l'une quelconque des revendications précédentes, **caractérisé en ce que** les faces (161, 162) du piston (157, 158) associées aux chambres de travail (A, B) de chaque unité piston-cylindre (155, 156) sont de grandeur différente et il est prévu une section transversale de dérivation (B), de préférence à l'intérieur du piston (157, 158), par laquelle un faible courant de volume de fuite s'écoule toujours en cas de différence de pression entre les chambres de travail (A, B) .

22. Système d'injection (100) selon au moins l'une quelconque des revendications précédentes, **caractérisé en ce que**, dans une position de purge du piston (157, 158) de l'unité piston-cylindre (155, 156), qui est de préférence une position de point mort du piston (157, 158), il existe une section transversale de dérivation de purge (EB) agrandie par rapport à la section transversale de dérivation (B), dans lequel la section transversale de dérivation de purge (EB) est formée de préférence par un élargissement de la section transversale du cylindre (163, 164) de l'unité piston-cylindre (155, 156).

23. Système d'injection (100) selon au moins l'une quelconque des revendications précédentes, **caractérisé en ce que** l'élargissement de la section transversale est effectué sous la forme d'un évidement et/ou d'un canal se trouvant dans la surface latérale intérieure du cylindre (163, 164) et enjambant la longueur axiale du piston (157, 158).

24. Système d'injection (100) selon au moins l'une quelconque des revendications précédentes, **caractérisé en ce qu'**une surface (165) du liquide hydraulique dans le réservoir hydraulique (106) est soumise à une dépression, de préférence à un vide, qui est produite et/ou qui est maintenue en continu au moyen d'une pompe à vide (166).

25. Procédé de régulation d'un système d'injection (100) pour l'injection de liquide dans un patient à l'intérieur d'un champ magnétique intense, d'un champ magnétique alternatif et/ou d'un champ électrique à haute fréquence, à utiliser en liaison avec un procédé médico-technique, comprenant une chambre protégée contre les champs électromagnétiques au moyen d'un blindage, dans lequel on délivre un liquide à injecter dans un patient par le déplacement d'un élément de transport à l'aide d'un dispositif d'entraînement (110) qui comprend un convertisseur de pression avec un piston mobile, dans lequel on réalise une production de pression au moyen d'une pompe hydraulique et une application de pression à un dispositif d'entraînement hydraulique (110), par des conduites hydrauliques (123 I, 123 II; 124 I, 124 II), **caractérisé en ce que** l'on détecte la position du piston du convertisseur de pression au moyen d'un détecteur de chemin parcouru, de préférence un potentiomètre, disposé dans la chambre protégée.

26. Procédé selon la revendication 25, **caractérisé en ce que** l'on effectue l'application de pression depuis l'extérieur de la chambre.

27. Procédé selon la revendication 25 ou 26, **caractérisé en ce que**, dans les périodes où le piston (157, 158) d'un convertisseur de pression (129, 130) doit être au repos, on régule le piston (157, 158) à la position d'arrêt respective, dans lequel on détecte la grandeur de régulation course du piston par un détecteur de course (127, 128) et on utilise comme grandeurs de réglage les pressions agissant dans les deux chambres de travail (A, B), dans lequel on maintient les deux pressions toujours au-dessus d'une pression minimale déterminée.

28. Procédé selon au moins l'une quelconque des revendications 25 à 27, **caractérisé en ce que** l'on maintient les pressions dans toutes les conduites hydrauliques toujours au-dessus d'une pression minimale déterminée, même pendant les périodes d'arrêt du piston respectif.

29. Procédé selon au moins l'une quelconque des revendications 25 à 28, **caractérisé en ce que** l'on utilise l'au moins une conduite hydraulique (123 I, 123 II; 124 I, 124 II) aussi comme conduite de commande pour la commande d'au moins une fonction déterminée de l'opération d'injection.

30. Procédé selon au moins l'une quelconque des revendications 25 à 29, **caractérisé en ce que** l'au moins une fonction est
a) une fonction de démarrage pour la délivrance du liquide,
b) une fonction d'arrêt pour arrêter le système d'injection,
c) une fonction de remplissage pour aspirer du liquide au moyen du dispositif de transport, et/ou
d) une fonction de purge pour purger l'élément de transport.

31. Procédé selon au moins l'une quelconque des revendications 25 à 30, **caractérisé en ce que** le procédé médico-technique est une tomographie par résonance magnétique.

32. Procédé selon au moins l'une quelconque des revendications 25 à 31, **caractérisé en ce que** l'on maintient en permanence un certain faible courant de volume à travers une section transversale de dérivation (B) à travers l'unité piston-cylindre (155, 156), qui est renvoyé au réservoir hydraulique (106), dans lequel une surface (165) du liquide hydraulique est soumise à une dépression, de préférence au moyen d'une pompe à vide (166).
